(19) Europäisches Patentamt
European Patent Office
Office européen des brevets

(11) EP 1 423 083 B1

(12) EUROPÄISCHE PATENTSCHRIFT

(45) Veröffentlichungstag und Bekanntmachung des Hinweises auf die Patenterteilung:
**12.09.2007 Patentblatt 2007/37**

(21) Anmeldenummer: **02797608.3**

(22) Anmeldetag: **22.08.2002**

(51) Int Cl.:
***A61K 8/35*** *(2006.01)* ***A61K 8/37*** *(2006.01)*
***A61K 8/60*** *(2006.01)* ***A61K 8/67*** *(2006.01)*
***A61Q 19/00*** *(2006.01)* ***A61Q 19/08*** *(2006.01)*

(86) Internationale Anmeldenummer:
**PCT/EP2002/009375**

(87) Internationale Veröffentlichungsnummer:
**WO 2003/020224 (13.03.2003 Gazette 2003/11)**

# (54) STABILISIERUNG OXIDATIONS-UND/ODER UV-EMPFINDLICHER WIRKSTOFFE

STABILISATION OF OXIDATION-SENSITIVE AND UV-SENSITIVE ACTIVE INGREDIENTS

STABILISATION DE PRINCIPES ACTIFS SENSIBLES A L'OXYDATION ET/OU AUX U.V.

(84) Benannte Vertragsstaaten:
**AT BE BG CH CY CZ DE DK EE ES FI FR GB GR IE IT LI LU MC NL PT SE SK TR**

(30) Priorität: **29.08.2001 DE 10141477**

(43) Veröffentlichungstag der Anmeldung:
**02.06.2004 Patentblatt 2004/23**

(73) Patentinhaber: **Beiersdorf AG**
**20245 Hamburg (DE)**

(72) Erfinder:
- **WENDEL, Volker**
  **20255 Hamburg (DE)**
- **GÖPPEL, Anja**
  **22527 Hamburg (DE)**

(56) Entgegenhaltungen:
**EP-A- 1 127 569**
**WO-A-01/41715**
**WO-A-01/52807**
**WO-A-02/17873**
**FR-A- 2 801 206**
**FR-A- 2 801 208**
**FR-A- 2 801 209**

- **BONDA C ET AL: "A NEW PHOTOSTABILIZER FOR FULL SPECTRUM SUNCREENS" COSMETICS & TOILETRIES, WHEATON, IL, US, Bd. 115, Nr. 6, 2000, Seiten 37-45, XP001010090 ISSN: 0361-4387**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 423 083 B1

## Beschreibung

Die vorliegende Erfindung betrifft Stoffkombinationen zur Stabilisierung oxidationsempfindlicher bzw. UV-empfindlicher Wirkstoffe sowie kosmetische und dermatologische Formulierungen mit auf diese Weise stabilisierten oxidationsempfindlichen bzw. UV-empfindlichen Wirkstoffen. Insbesondere betrifft sie kosmetische und dermatologische Lichtschutzformulierungen und Formulierungen mit UV-empfindlichen Lichtschutzfiltersubstanzen, die durch den Einsatz dieser Stoffkombinationen stabilisiert werden.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons sowie des Triazins handelt.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hier durch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, daß kosmetische und dermatologische Lichtschutzzubereitungen sowohl gegen UV-B- als auch gegen UV-A-Strahlung ausreichenden Schutz bieten.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen.

Die Einsatzkonzentration bekannter als Feststoff vorliegender Lichtschutzfiltersubstanzen ist allerdings häufig - gerade in Kombination mit anderen zu lösenden Substanzen - begrenzt. Es bereitet daher gewisse formulierungstechnische Schwierigkeiten, höhere Lichtschutzfaktoren bzw. UV-A-Schutzleistung zu erzielen.

Vorteilhafte UV-A-Filtersubstanzen sind z. B. Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Der Hauptnachteil aller im UV-Bereich absorbierenden Dibenzoylmethanderivate ist eine gewisse Instabilität gegenüber UV-Strahlung, so daß diese Komponenten unter UV-Einfluß zu inaktiven Produkten zersetzt werden und für die UV-Absorption nicht mehr zur Verfügung stehen. Zubereitungen des Standes der Technik mit einem Gehalt an diesen Substanzen enthalten daher zweckmäßigerweise auch bestimmte UV-Stabilisatoren, wie beispielsweise Ethylhexyl-2-cyano-3,3-diphenylacrylat (Octocrylen) oder 4-Methylbenzylidencampher.

Es war eine Aufgabe der vorliegenden Erfindung, die Nachteile des Standes der Technik zu beseitigen und auf einfache Weise zu Zubereitungen zu gelangen, welche sich durch eine hohe UV-, insbesondere UV-A-Schutzleistung auszeichnen und in welchen auf den Einsatz üblicher UV-Stabilisatoren verzichtet werden kann.

Der Stand der Technik kennt ferner eine Reihe von verschiedenen effizienten, lipophilen Hautpflegewirkstoffen - wie beispielsweise Ubichinone, Retinoide und Carotinoide - die ungesättigte, aromatische oder benzoide Strukturelemente enthalten; deren Einsatz in kosmetischen und dermatologischen Formulierungen, insbesondere in Formulierungen vom Typ Öl-in-Wasser, sehr wünschenswert ist. Unglücklicherweise sind derartige Substanzen aber häufig sehr

instabil, so daß sie insbesondere in wäßrigen Medien schnell zerfallen und auf diese Weise ihre Wirksamkeit verlieren.

Eine weitere Aufgabe der vorliegenden Erfindung war es daher, die Stabilität von oxidationsempfindlichen bzw. UV-empfindlichen Wirkstoffen zu erhöhen sowie stabile Zubereitungen mit oxidationsempfindlichen bzw. UV-empfindlichen Wirkstoffen zu schaffen, deren Wirksamkeit über einen langen Zeitraum erhalten bleibt.

Die Veröffentlichung C. Bonda, D.C. Steinberg, A New Photostabilizer for Full Sprectrum Sunscreens, XP001010090, beschreibt den UV-Stabilisator HallbiteTQ für oxidations- oder UV-empfindliche Wirkstoffe wie z. B. UV-Filter. Über den vorteilhaften Einsatz von Triglyeridwachsen wird allerdings nichts offenbart.

FR 2 801 206 offenbart kosmetische Zubereitungen, insbesondere Sonnenschutzzubereitungen, die mindestens ein Derivat der Naphthalenmono- oder polycarbonsäure und einen UV-Fitter vom Typ Hydroxiyphenylbenzotriazolderivat. Über den Einsatz von Triglyceridwachsen wird auch hier nichts offenbart

Die Schrift FR 2 801 209 offenbart eine kosmetische Emulsion ohne Emulgator, die eine wässrige Phase, eine Fettphase, einen UV-Filter und ein Derivat der Naphtalinmono- oder polycarbonsäure enthält Auch hier wird nichts über den Einsatz von Triglyceridwachsen zur UV-Stabilisierung offenbart.

Die Schrift EP 1 127 569 offenbart eine lichtschutzwirksame Wirkstoffkombination aus UV-Filtersubstanzen und die Alkylnaphthalaten. Über einen Einsatz von Triglyceridwachsen wird auch hier nichts offenbart.

Es war überraschend und für den Fachmann nicht vorauszusehen, daß kosmetische und dermatologische Formulierungen mit mindestens einem oxidations- und/oder UV-empfindlichen Wirkstoff, dadurch gekennzeichnet, daß sie

(a) mindestens ein Dialkylnaphthalat, welches sich durch die Strukturformel

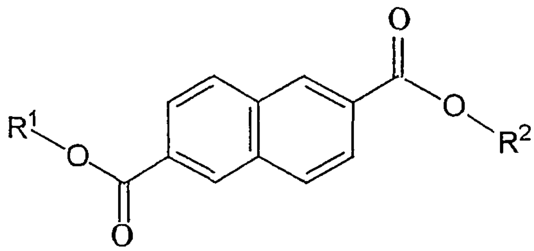

auszeichnet,
worin $R^1$ und $R^2$ unabhängig voneinander gewählt werden aus der Gruppe der verzweigten und unverzweigten Alkylgruppen mit 6 bis.24 Kohlenstoffatomen, und
(b) mindestens ein Wachs welches aus der Gruppe enthalten, der Triglyceride gewählt wird,

den Nachteilen des Standes der Technik abhelfen.

Liegen der oder die oxidations- und/oder UV-empfindlichen Wirkstoffe in einer erfindungsgemäßen Formulierung vor, so sind sie gegen die durch UV-Strahlung induzierte Zersetzung in hervorragender Weise geschützt. Dieses gilt insbesondere für Dibenzoylmethanderivate.

Gegenstand der Erfindung ist daher auch die
Verwendung von Stoffkombinationen, welche

(a) mindestens ein Dialkylnaphthalat, welches sich durch die Strukturformel

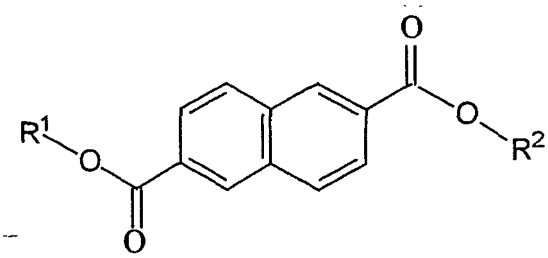

auszeichnet,
worin $R^1$ und $R^2$ unabhängig voneinander gewählt werden aus der Gruppe der verzweigten und unverzweigten Alkylgruppen mit 6 bis 24 Kohlenstoffatomen, und

(b) mindestens ein Wachs, welches aus der Gruppe der Triglyceride gewählt wird, enthalten,

zur Stabilisierung kosmetischer oder dermatologischer Wirkstoffe gegen die durch UV-Strahlung induzierte Zersetzung.

Die Zubereitungen im Sinne der vorliegenden Erfindung können bevorzugt neben einer oder mehrerer Ölphasen zusätzlich eine oder mehrere Wasserphasen enthalten und beispielsweise in Form von W/O-, O/W-, W/O/W- oder O/W/O-Emulsionen vorliegen. Solche Formulierungen können vorzugsweise auch eine Mikroemulsion, eine PIT-Emulsion, eine wasserhaltige oder wasserfreie Sitftformulierung, eine Feststoff-Emulsionen (d. h. eine Emulsion, welche durch Feststoffe stabilisiert ist, z. B. eine Pickering-Emulsion), eine sprühbare Emulsion oder eine Hydrodispersion sein.

Die erfindungsgemäßen Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität bei gleichzeitig hervorragenden Hautpflegedaten aus.

Es war insbesondere überraschend, daß bei der Verwendung gemäß der vorliegenden Erfindung gänzlich auf den Einsatz weiterer UV-Stabilisatoren, insbesondere auf den Einsatz von Ethylhexyl-2-cyano-3,3-diphenylacrylat (Octocrylen) oder 4-Methylbenzylidencampher verzichtet werden kann.

Ferner läßt sich durch die erfindungsgemäße Verwendung überraschend die Stabilität von lipophilen Wirkstoffen in wasserhaltigen kosmetischen oder dermatologischen Formulierungen, insbesondere in O/W-Formulierungen, gegenüber dem Stand der Technik erheblich steigern.

Gegenstand der Erfindung ist daher auch die
Verwendung von Stoffkombinationen, welche

(a) mindestens ein Dialkylnaphthalat, welches sich durch die Strukturformel

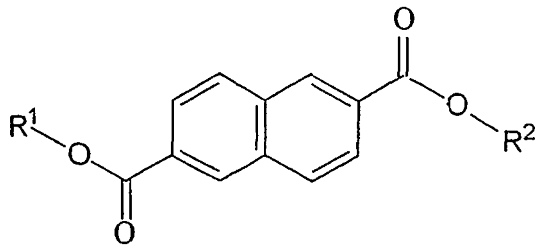

auszeichnet,
worin $R^1$ und $R^2$ unabhängig voneinander gewählt werden aus der Gruppe der verzweigten und unverzweigten Alkylgruppen mit 6 bis 24 Kohlenstoffatomen, und
(b) mindestens ein Wachs, welches aus der Gruppe der Triglyceride ausgewählt wird,

enthalten, zur Verbesserung der Wirksamkeit und zur Erhöhung der Stabilität von lipophilen Wirkstoffen in kosmetischen oder dermatologischen Zubereitungen.

Vorteilhafte lipophile Wirkstoffe, welche durch die erfindungsgemäße Verwendung in hervorragender Weise stabilisiert werden, sind solche, deren log P-Wert größer als 3,5 ist. P ist der Verteilungskoeffizient, welcher definiert ist als das Verhältnis der Gleichgewichtskonzentrationen einer gelösten Substanz in einem Zwei-Phasen-System, welches aus zwei miteinander im wesentlichen nicht mischbaren Lösungsmitteln besteht. Diese beiden Lösungsmittel sind im vorliegenden Fall n-Octanol und Wasser, d. h.

$$P_{ow} = \frac{c_{n\text{-}Octanol}}{c_{Wasser}}$$

Es ist vorteilhaft im Sinne der vorliegenden Erfindung, den oder die lipophile(n) Wirkstoffe aus der Gruppe der Ubichinone und Plastochinone zu wählen. Ganz besonders vorteilhaft im Sinne der vorliegenden Erfindung ist Coenzym Q10, welches einen log P-Wert von etwa 15 hat.

Es war insbesondere überraschend, daß sich sehr vorteilhafte Zubereitungen gemäß der vorliegenden Erfindung erhalten lassen, wenn der oder die Wirkstoff(e) nur aus der Gruppe der Ubichinone ausgewählt wird/werden.

Weitere erfindungsgemäß vorteilhafte lipophile Wirkstoffe sind Retinoide (Vitamin A-Säure und/oder deren Derivate) bzw. Vitamin A und/oder dessen Derivate. In die Gruppe der erfindungsgemäß vorteilhaften Retinoide werden begrifflich alle kosmetisch und/oder pharmazeutisch unbedenklichen Retinoide, einschließlich des Retinols und seiner Ester, des Retinals sowie der Retinoësäure (Vitamin A-Säure) und deren Ester einbezogen. Besonders vorteilhaft im Sinne der vorliegenden Erfindung sind Retinol (mit einem log P-Wert von etwa 7) und Retinylpalmitat (mit einem log P-Wert von etwa 13).

Es war ferner insbesondere überraschend, daß sich sehr vorteilhafte Zubereitungen gemäß der vorliegenden Erfindung erhalten lassen, wenn der oder die Wirkstoff(e) nur aus der Gruppe der Retinoide ausgewählt wird/werden.

Weitere erfindungsgemäß vorteilhafte lipophile Wirkstoffe sind Carotinoide. Besonders vorteilhaft im Sinne der vorliegenden Erfindung ist beispielsweise das β-Carotin, welches einen log P-Wert von 15 hat.

Weitere erfindungsgemäß vorteilhafte lipophile Wirkstoffe sind:

- Liponsäure und Derivate,
- Vitamin E und Derivate,
- Vitamin F,
- Dioic Acid [8-Hexadecen-1,16-dicarbonsäure (CAS-Nummer 20701-68-2)]

Die Menge an lipophilen Wirkstoffen (eine oder mehrere Verbindungen) in den Zubereitungen beträgt vorzugsweise 0,0001 bis 10 Gew.-%, besonders bevorzugt 0,001 bis 5 Gew.-%, bezogen auf das Gesamtgewicht der Zubereitung.

Vorteilhaft im Sinne der vorliegenden Erfindung sind Dialkylnaphthalate, für die $R^1$ und/oder $R^2$ verzweigte Alkylgruppen mit 6 bis 10 Kohlenstoffatomen darstellen. Ganz besonders bevorzugt im Sinne der vorliegenden Erfindung ist Diethylhexylnaphthalat, welches beispielsweise unter der Handelsbezeichnung Hallbrite TQ™ von CP Hall oder Corapan TQ™ von H&R erhältlich ist.

Erfindungsgemäß vorteilhaft enthalten kosmetische oder dermatologische Zubereitungen 0,001 bis 20 Gew.-%, vorzugsweise 0,01 bis 15 Gew.-%, ganz besonders bevorzugt 1 bis 15 Gew.-% eines oder mehrerer Dialkylnaphthalate.

<u>Wachse:</u>

Nach Festlegung der Deutschen Gesellschaft für Fettwirtschaft (Fette, Seifen, Anstrichmittel, 76, 135 [1974*]*) werden zur Kennzeichnung des Begriffes "Wachs" in der Regel die mechanisch-physikalischen Eigenschaften der Wachse, welche für ihre Verwendung maßgebend sind, herangezogen, während für die Begriffsbestimmung die jeweilige chemische Zusammensetzung unberücksichtigt bleibt.

"Wachs" ist - ähnlich wie "Harz"- eine Sammelbezeichnung für eine Reihe natürlicher oder künstlich gewonnener Stoffe, die in der Regel folgende Eigenschaften aufweisen: bei 20 °C knetbar, fest bis brüchig hart, grob- bis feinkristallin, durchscheinend bis opak, jedoch nicht glasartig, über 40 °C ohne Zersetzung schmelzend, schon wenig oberhalb des Schmelzpunkts verhältnismäßig niedrigviskos und nicht fadenziehend, stark temperaturabhängige Konsistenz und Löslichkeit und unter leichtem Druck polierbar. Ist in Grenzfällen bei einem Stoff mehr als eine der vorstehend genannten Eigenschaften nicht erfüllt, so ist er kein Wachs im Sinne dieser Definition. Wachse unterscheiden sich von ähnlichen synthetischen oder natürlichen Produkten (z. B. Harzen, plastischen Massen usw.) hauptsächlich darin, daß sie in der Regel etwa zwischen 30 und 90 °C, in Ausnahmefällen auch bis zu etwa 200 °C, in den schmelzflüssigen, niedrigviskosen Zustand übergehen und praktisch frei von aschebildenden Verbindungen sind.

Wachse im Sinne der vorliegenden Erfindung sind Verbindungen, welche dadurch charakterisiert sind, daß sie zusammen mit den anderen Ölkomponenten der erfindungsgemäßen Zubereitungen (wie beispielsweise polare, flüssige Verbindungen, UV-Filter und deren Lösungsmittel usw.) eine bei Raumtemperatur streichfähige oder fließfähige Masse bilden, welche bei 20 °C eine Viskosität von mehr als 500 mPa·s aufweist.

Auch Pflanzenwachse sind vorteilhaft im Sinne der vorliegenden Erfindung, wie beispielsweise Sesamwachs, Jojobaöl und dergleichen mehr.

Die Wachskomponenten sind aus der Gruppe der Triglyceride zu wählen. Besonders vorteilhaft sind die im folgenden aufgelisteten Triglyceride:

| **Glycerid** | **Handelsname** | **erhältlich bei** |
|---|---|---|
| $C_{16-18}$ Triglycerid | Cremeol HF-52-SPC | Aarhus Oliefabrik |
| Hydrierte Coco-Glyceride | Softisan 100 | Hüls AG |
| Caprylic/Capric/Isostearic/Adipic Triglycerid | Softisan 649 | Dynamit Nobel |
| $C_{18-36}$ Triglycerid | Syncrowax HGLC | Croda GmbH |

(fortgesetzt)

| Glycerid | Handelsname | erhältlich bei |
|---|---|---|
| Glyceryltribehenat | Syncrowax HRC | Croda GmbH |
| Glyceryl-tri-(12-hydroxystearat) | Thixcin R | Rheox / NRC |
| Hydriertes Ricinusöl | Cutina HR | Henlek KGaA |
| $C_{16-24}$ Triglycerid | Cremeol HF-62-SPC | Aarhus Oliefabrik |

Besonders bevorzugt im Sinne der vorliegenden Erfindung ist auch Sheabutter, auch Karitéfett oder Galambutter genannt (CAS-Nr. 68920-03-6). Sheabutter ist das Fett der Samen bzw. Kerne der Familie der Sapotaceae angehörenden Pflanze Butyrospermum Parkii, das zu etwa 34 bis 45 Gew.-% aus festen Fettsäuren (vornehmlich Stearinsäure) und zu etwa 50 bis 60 Gew.-% aus flüssigen Fettsäuren (vornehmlich Ölsäure enthaltend) besteht.

Die Gesamtmenge an einem oder mehreren Wachsen und/oder Ölverdickungsmitteln in den fertigen kosmetischen oder dermatologischen Zubereitungen wird vorteilhaft aus dem Bereich von 0,1 bis 30,0 Gew.-%, bevorzugt zwischen 0,5 und 15 Gew.-% gewählt, bezogen auf das Gesamtgewicht der Zubereitungen.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikon-derivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate sowie D-Biotin, natürliche und/oder synthetische Isoflavonoide, alpha-Glucosylrutin, Panthenol, Aloe Vera.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Vorteilhafte weitere Wirkstoffe sind natürliche Wirkstoffe und/oder deren Derivate, wie z. B. Phytoen, Carnitin, Carnosin, Kreatin, Taurin und/oder β-Alanin.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Ferner vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/$C_{10-30}$ Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Vorteilhaft sind Verbindungen, die die INCI-Bezeichnung Ammoniumacryloyldimethyltaurate/Vinylpyrrolidoncopolymere tragen.

Erfindungsgemäß vorteilhaft weisen das oder die AmmoniumacryloyldimethyltaurateNinylpyrrolidoncopolymere die Summenformel $[C_7H_{16}N_2SO_4]_n[C_6H_9NO]_m$ auf, einer statistischen Struktur wie folgt entsprechend

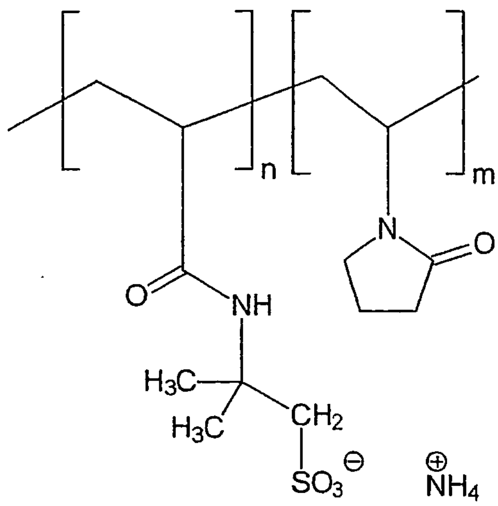

Bevorzugte Spezies im Sinne der vorliegenden Erfindung sind in den Chemical Abstracts unter den Registratumummern 58374-69-9, 13162-05-5 und 88-12-0 abgelegt und erhältlich unter der Handelsbezeichnung Aristoflex® AVC der Gesellschaft Clariant GmbH.

Vorteilhaft sind ferner Copolymere/Crosspolymere umfassend Acryloyldimethyl Taurate, wie beispielsweise Simugel ® EG oder Simugel ® EG von der Gesellschaft Seppic S.A.

Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden.

Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat Aluminium-bzw. Natrium-Stärke Octenylsuccinat und dergleichen), Pigmente, die weder hauptsachlich UV-Filter noch färbende Wirkung haben (wie z. B. Bomitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol* CC bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, $C_{12-13}$-Alkyllactat, Di-$C_{12-13}$-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an $C_{12-15}$-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen

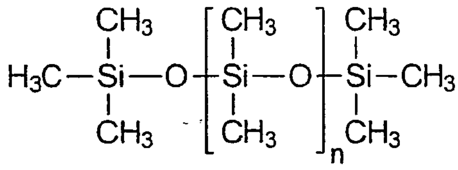

werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz und/oder ein oder mehrere Silikonderivate als weitere Phase enthalten. Ölfreie Formulierungen im Sinne der vorliegenden Erfindung können vorteilhaft auch weitere lipophile Komponenten - wie beispielsweise lipophile Wirkstoffe - enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate).

Bevorzugte anorganische Pigmente sind Metalloxide und/oder andere in Wasser schwerlösliche oder unlösliche Metallverbindungen, insbesondere Oxide des Titans ($TiO_2$) Zinks (ZnO), Eisens (z. B. $Fe_2O_3$), Zirkoniums ($ZrO_2$) Siliciums ($SiO_2$) Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| Zinkoxid Neutral | / | H&R |
| MZ 303 M | 5% Methicon | Tayca Corp. |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |
| Tioveil AQ 10PG | Alumina / Silica | Solaveil / Uniquema |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.

- 2,4-Bis-{[4-(2-Ethyl-hexyloxy)-2-hydroxy]-phenyl}-6-(4-methoxyphenyl)-1,3,5-triazin (INCI: Methylene Bis-Benzotriazolmethylbutylphenol), welches unter der Handelsbezeichnung Tinosorb® S bei der CIBA-Chemikalien GmbH erhältlich ist;
- Dioctylbutylamidotriazon (INCI: Diethylhexylbutamidotriazone), welches unter der Handelsbezeichnung UVASORB HEB bei Sigma 3V erhältlich ist;
- 4,4',4''-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Bisoctyltriazol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches unter der Handelsbezeichnung Mexoryl® XL bei der Fa. Chimex erhältlich ist.

Die weiteren UV-Filtersubstanzen können öllöslich sein. Vorteilhafte öllösliche Filtersubstanzen sind z. B.:

- ■ 3-Benzylidencampher-Derivate, vorzugsweise 3-(4-Methylbenzyliden)campher, 3-Benzylidencampher;
- ■ 4-Aminobenzoesäure-Derivate, vorzugsweise 4-(Dimethylamino)-benzoesäure(2-ethylhexyl)ester, 4-(Dimethylamino)benzoesäureamylester;
- ■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- ■ sowie an Polymere gebundene UV-Filter.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Ferner kann es gegebenenfalls von Vorteil sein, Filmbildner in die erfindungsgemäßen kosmetischen oder dermatologischen Zubereitungen einzuarbeiten, beispielsweise um die Wasserfestigkeit der Zubereitungen zu verbessern oder die UV-Schutzleistung zu erhöhen (UV-A- und/oder UV-B-Boosting). Geeignet sind sowohl wasserlösliche bzw. dispergierbare als auch fettlösliche Filmbildner, jeweils einzeln oder in Kombination miteinander.

Vorteilhafte wasserlöslich bzw. dispergierbare Filmbildner sind z. B. Polyurethane (z. B. die Avalure® -Typen von Goodrich), Dimethicone Copolyol Polyacrylate (Silsoft Surface® von der Witco Organo Silicones Group), PVPNA (VA = Vinylacetat) Copolymer (Luviscol VA 64 Powder der BASF) etc.

Vorteilhafte fettlösliche Filmbildner sind z. B., die Filmbildner aus der Gruppe der Polymere auf Basis von Polyvinylpyrrolidon (PVP)

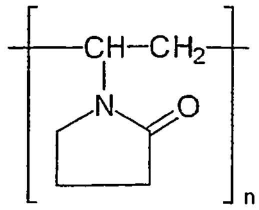

Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind, sowie das Tricontayl PVP und dergleichen mehr.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispiele:**

**1. O/W Sonnenschutz Emulsionen**

| | 1 | 2 | 3 | 4 | 5 | 6 | 7 |
|---|---|---|---|---|---|---|---|
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | | 2,00 | | | |
| PEG-40 Stearat | 0,50 | | | | | 2,00 | |
| PEG-100 Stearat | | | 4,50 | | | 1,50 | 0,75 |
| Stearyl Alkohol | | | 3,00 | | | 2,00 | 0,50 |
| Cetyl Alkohol | 2,50 | 1,00 | | 1,50 | 0,50 | | 2,00 |
| Ethylhexyl Methoxycinnamat | | | | 5,00 | 6,00 | | 8,00 |
| Aniso Triazin | | | 3,00 | 2,00 | | | 2,50 |
| Butyl Methoxydibenzoylmethan | | | | 1,50 | 2,80 | 2,00 | 1.50 |
| Bisimidazylat | 2,50 | | | | 1,00 | | 0,30 |
| Ethylhexyl Triazon | 4,00 | | 3,00 | 4,00 | 4,00 | 2,00 | |
| 4-Methylbenzyliden Campher | 4,00 | | | | 2,00 | 4,00 | 2,00 |
| Octocrylen | | | 7,50 | | | | 2,50 |
| Diethylhexyl Butamido Triazon | 1,00 | | 1,00 | | 1,00 | | |
| Phenylbenzmidazol Sulfonsäure | 0,50 | | | 3,00 | | | |
| Bisoctyltriazol | 2,00 | | 0,50 | 1,50 | 2,50 | | |
| Homosalat | | | | | | | |
| Ethylhexylsalicylat | | | 3,00 | | | | 5,00 |
| Drometrizol Trisiloxan | | | 0,5 | | | 1,00 | |
| Terephthaliden Dicamphor Sulfonsäure | | | | | 1,00 | 0,50 | |
| Diethylhexyl-2,6-naphthalat | 3,50 | 4,80 | 7,00 | 9,50 | 6,70 | 5,50 | 8,00 |
| Titandioxid MT-100Z | 1,00 | | | | 2,00 | 2,00 | |

(fortgesetzt)

| | 1 | 2 | 3 | 4 | 5 | 6 | 7 |
|---|---|---|---|---|---|---|---|
| Zinkoxid HP1 | | | 1,50 | 1,00 | | 2,00 | 3,00 |
| C12-15 Alkyl Benzoat | | 2,50 | | | 4,00 | 7,00 | 5,00 |
| Dicaprylyl Ether | | | 3,50 | | 2,00 | | |
| Butylenglycol Dicaprylat/Dicaprat | 5,00 | | | 6,00 | | | |
| Dicaprylyl Carbonat | | | 6,00 | | | 2,00 | 2,00 |
| Dimethicon | | 0,50 | 1,00 | | 2,00 | | |
| Cyclomethicon | 2,00 | | | 0,50 | | | 0,50 |
| Ethyl Galaktomannan (N-Hance® AG200) | | 3,50 | | 2,00 | | | 1,00 |
| $C_{18-36}$ Triglycerid | 2,00 | | 5,50 | 3,00 | | | |
| Hydrogenated Cocoglyceride | | | | | 4,50 | 3,00 | 2,00 |
| Shea Butter | | 2,00 | | | | | 0,50 |
| PVP Hexadecen Copolymer | 0,50 | | | 0,50 | 1,00 | | 1,00 |
| Tricontanyl PVP | | 0,50 | 1,00 | | | | 1,00 |
| Glycerin | 3,00 | 7,50 | | 7,50 | 5,00 | | 2,50 |
| Xanthan Gummi | 0,15 | | 0,05 | | | | 0,30 |
| Sodium Carbomer | | 0,20 | 0,10 | 0,20 | | | |
| Vitamin E | | 0,50 | 1,00 | | | | 0,25 |
| Alpha-Glucosilrutin | | | 0,10 | 0,45 | 0,12 | 0,30 | 0,10 |
| Vitamin A | 0,50 | | | | 0,75 | | 1,00 |
| DMDM Hydantoin | | 0,60 | 0,40 | 0,20 | | | |
| Konkaben LMB ® | | | | 0,18 | 0,20 | 0,10 | 0,15 |
| Methylparaben | 0,15 | | 0,25 | | 0,50 | | |
| Phenoxyethanol | 1,00 | 0,40 | | 0,40 | 0,50 | 0,40 | 0,60 |
| Ethanol | | 2,00 | 1,50 | | 3,00 | | 1,00 |
| Parfüm | 0,20 | 0,20 | 0,20 | 0,20 | 0,20 | 0,20 | 0,20 |
| Wasser | ad 100 | ad 100 | ad 100 | ad 100 | ad 100 | ad 100 | ad 100 |

**2. Hydrodispersionen**

| | 1 | 2 | 3 | 4 | 5 * |
|---|---|---|---|---|---|
| Ceteareth-20 | 1,00 | | | 0,5 | |
| Cetyl Alkohol | | | 1,00 | | |
| Sodium Carbomer | | 0,20 | | 0,30 | |
| Acrylates/C10-30 Alkyl Acrylat Crosspolymer | 0,50 | | 0,40 | 0,10 | 0,10 |
| Xanthan Gummi | | 0,30 | 0,15 | | 0,50 |
| Ethylhexyl Methoxycinnamat | | | | 5,00 | |
| Aniso Triazin | | 1,50 | | 2,00 | |

(fortgesetzt)

| | 1 | 2 | 3 | 4 | 5 * |
|---|---|---|---|---|---|
| Butyl Methoxydibenzoylmethan | 1,00 | 0.50 | | 3.00 | |
| Bisimidazol | 0,50 | | | 2,00 | |
| Ethylhexyl Triazon | 4,00 | | 3,00 | 4,00 | |
| Octocrylen | | 4,00 | 3,90 | | |
| Diethyhexyl Butamido Triazon | 1,00 | | | 2,00 | |
| Phenylbenzmidazol Sulfonsäure | 0,50 | | | 3,00 | |
| Bisoctyltriazol | 2,50 | 0,50 | | | |
| Drometrizol Trisiloxan | | | 1,00 | | |
| Diethylhexyl-2,6-naphthalat | 4,50 | 8,00 | 7,20 | 5,50 | 14,00 |
| Titandioxid MT-100Z | 0,50 | | 2,00 | | |
| Zinkoxid HP1 | | | 1,00 | 2,00 | |
| $C_{18-36}$ Triglycerid | 3,50 | 2,50 | | | |
| Glyceryl-tri-(12-hydroxystearat) | | | 4,00 | | |
| Sheabutter | | | 2,50 | 4,50 | |
| C12-15 Alkyl Benzoat | 2,00 | 2,50 | | | |
| Cocoglyceride (Myritol® 331) | | 4,00 | | | |
| Butylenglycol Dicaprylat/Dicaprat | 4,00 | | 2,00 | 6,00 | |
| Dicaprylyl Carbonat | | 2,00 | 6,00 | | |
| Dimethicon | | 0,50 | 1,00 | | |
| Phenyltrimethicon | 2,00 | | | 0,50 | 2,00 |
| PVP Hexadecen Copolymer | 0,50 | | | 0,50 | 1,00 |
| Tricontanyl PVP | 0,50 | | 1,00 | | |
| Ethylhexylglycerin | | | 1,00 | . | 0,50 |
| Glycerin | 3,00 | 7,50 | | 7,50 | 2,50 |
| Glycin Soja | | | 1,50 | | |
| Vitamin E | 0,50 | | 0,25 | | 1,00 |
| Alpha-Glucosylrutin | | | 0,35 | 0,50 | |
| DMDM Hydantoin | | 0,60 | 0,40 | 0,20 | |
| Konkaben LMB ® | 0,20 | | | | 0,15 |
| Methylparaben | 0,50 | | 0,25 | 0,15 | |
| Phenoxyethanol | 0,50 | 0,40 | | 1,00 | 0,60 |
| Fucogel®-1000 | | | | | |
| Ethanol | 3,00 | 2,00 | 1,50 | | 1,00 |
| Farbstoffe, wasserlöslich | | | | 0,12 | |
| Parfüm | 0,20 | 0,20 | 0,20 | 0,20 | 0,20 |
| Wasser | ad 100 | ad 100 | ad 100 | ad 100 | ad 100 |

**3. W/O Sonnenschutz Emulsionen**

| | 1 | 2 | 3 | 4 | 5* |
|---|---|---|---|---|---|
| Cetyldimethicon Copolyol | | 2,50 | | 4,00 | |
| Laurylmethicon Copolyol | | | 1,50 | | |
| Polyglyceryl-2-dipolyhydroxystearat | 5,00 | | | | 4,50 |
| PEG-30-dipolyhydroxystearat | | | 5,00 | | |
| Ethylhexyl Methoxycinnamat | | 8,00 | | 5,00 | 4,00 |
| Aniso Triazin | 2,00 | 2,50 | | 2,00 | 2,50 |
| Butyl Methoxydibenzoylmethan | | 3,00 | 2,00 | 1,00 | |
| Bisimidazylat | | | | 2,00 | 3,00 |
| Ethylhexyl Triazon | | | 3,00 | 4,00 | |
| Octocrylen | 0,90 | 2,50 | 3,90 | | 2,50 |
| Diethyhexyl Butamido Triazon | 1,00 | | | 2,00 | |
| Phenylbenzmidazol Sulfonsäure | 0,50 | | | 3,00 | 2,00 |
| Bisoctyltriazol | | | 2,00 | 0,50 | |
| Drometrizol Trisiloxan | | 1,00 | 0,25 | 2,00 | |
| Terephthaliden Dicamphor Sulfonsäure | | | 1,00 | | |
| Diethylhexyl-2,6-naphthalat | 8,00 | 3,50 | 10,00 | 8,50 | 12,00 |
| Titandioxid T805 | | 2,00 | 1,50 | | 3,00 |
| Z-Cote®HP1 1 | 1,00 | | 3,00 | | 7,00 |
| Mineralöl | | | 10,0 | | 8,00 |
| C12-15 Alkyl Benzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| Bentone ® 38 | 4,00 | | | | |
| Glyceryltribehenat | | 3,50 | | 2,50 | |
| Siliconyl Beeswachs | | 1,25 | | | 6,00 |
| Carnaubawachs | 3,00 | | | | |
| Ethyl Galaktomannan | | | 3,50 | 5,00 | |
| PVP Hexadecen Copolymer | 0,50 | | | 0,50 | 1,00 |
| Tricontanyl PVP | | | 0,50 | 1,00 | 0,50 |
| Ethylhexylglycerin | | 0,30 | 1,00 | | 0,50 |
| Glycerin | 3,00 | 7,50 | | 7,50 | 2,50 |
| Glycine Soja | | 1,00 | 1,50 | | |

(fortgesetzt)

| | 1 | 2 | 3 | 4 | 5* |
|---|---|---|---|---|---|
| $MgSO_4$ | 1,00 | 0,50 | | 0,50 | |
| Vitamin E Acetat | 0,50 | | 0,25 | | 1,00 |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | 0,60 |
| Ethanol | 3,00 | | 1,50 | | 1,00 |
| Parfüm | 0,20 | 0,20 | 0,20 | 0,20 | 0,20 |
| Wasser | ad 100 | ad 100 | ad 100 | ad 100 | ad 100 |

**4. Feststoffstabilisierte Emulsionen**

| | 1* | 2 | 3 | 4* | 5 |
|---|---|---|---|---|---|
| Mineralöl | | | | | 16,0 |
| Octyldodecanol | 6,0 | | 7.5 | 7.5 | 5,0 |
| Caprylic/Capric Triglycerid | | | | | 6,0 |
| C12-15- Alkyl Benzoat | 7,0 | 8,0 | 7,5 | 7,5 | |
| Butylen Glycol Dicaprylat/Dicaprat- | 4,0 . | 8,0 | | | |
| Dicaprylyl Ether | | 8,0 | 7,5 | 7,5 | |
| Dicaprylyl Carbonat | 4,0 | | | | |
| Hydroxyoctacosanyl Hydroxystearat | 2,0 | | 2,0 | 2,0 | 1,5 |
| PVP/Hexadecen Copolymer | | | | 1,0 | 0,7 |
| Disteardimonium Hectorit | 1,0 | 1,0 | | 0,5 | 1,0 |
| Dimethicon | | 2,0 | | | |
| Cyclomethicon | | | | 2,0 | |
| Ethylhexylmethoxycinnamat | 5,0 | | 5,0 | | |
| Butyl Methoxydibenzoylmethan | 3.00 | 2,0 | 0.50 | 1.80 | 1,0 |
| 4-Methylbenzyliden Camphor | | 4,0 | | | 2,0 |
| Ethylhexyltriazon | 2,0 | 2,0 | | | 1,0 |
| Bisoctyltriazol | | 3,00 | | 2,50 | |
| Aniso Triazin | 2,5 | | 2,5 | | |
| Eusolex T-2000 | 1,5 | | 4,0 | 0,5 | 1,5 |
| Titandioxid T 805 | | | 2,0 | | |
| Zinkoxid NDM | | 4,5 | 2,0 | | |
| Phenylbenzimidazol Sulfonsäure | 2,0 | | | | |
| Bisimidazylat | | 1,00 | | 1,50 | 3,00 |
| Bornitrid | | | | | 0,5 |
| $C_{18-36}$ Triglycerid | | | 4,5 | | 1,5 |

(fortgesetzt)

| | 1* | 2 | 3 | 4* | 5 |
|---|---|---|---|---|---|
| Hydrogenated Cocoglyceride | | 2,5 | | | |
| $C_{20-40}$ Alkylstearat | 3,5 | | | 5,0 | |
| Stärke/-Natriummetaphosphat-Polymer | 0,5 | | 1,5 | | |
| Korn Stärke Modifiziert | | 1,0 | | | |
| Acrylat Copolymer | | | | 0,25 | |
| Talk | | | | 2,0 | |
| Natrium Chlorid | 1,0 | 1,0 | 1,0 | | |
| Diethylhexyl-2,6-naphthalat | 4,00 | 6,50 | 7,50 | 9,50 | 5,00 |
| Polyurethan | | 0,50 | 1,50 | | 0,40 |
| Magnesium Sulfat | | | | | 0,70 |
| Natronlauge 45% | 0,5 | 0,5 | | | |
| Glycerin | 5,0 | 7,5 | 5,0 | 10,0 | 3,0 |
| Trinatrium EDTA | | 1,0 | 1,0 | | 1,0 |
| Propylen Carbonat | 0,33 | 0,33 | 0,33 | | 0,33 |
| Methylparaben | 0,21 | 0,21 | 0,2 | 0,2 | 0,21 |
| Propylparaben | 0,07 | 0,07 | | | 0,07 |
| Phenoxyethanol | 0,5 | 0,5 | 0,5 | 0,5 | 0,5 |
| Hexamidin Diisethionat | | | 0,08 | 0,08 | |
| Alcohol | | 5,0 | | | |
| Parfüm | 0,15 | | | 0,50 | 0,35 |
| Wasser | ad 100 | ad 100 | ad 100 | ad 100 | ad100 |

**5. Stifte**

| | 1 | 2 | 3 | 4* |
|---|---|---|---|---|
| Caprylic/Capric Triglycerid | 12 | 10 | 6 | |
| Octyldodecanol | 7 | 14 | 8 | 3 |
| Butylene Glycol Dicaprylat/Dicaprat | | | | 12 |
| Pentaerythrityl Tetraisostearat | 10 | 6 | 8 | 7 |
| Polyglyceryl-3 Diisostearat | 2,5 | | | |
| Bis-Diglyceryl Polyacyladipate-2 | 9 | 8 | 10 | 8 |
| Cetearyl Alcohol | 8 | 11 | 9 | 7 |
| Myristyl Myristate | 3,5 | 3 | 4 | 3 |
| Beeswax | 5 | 5 | 6 | 6 |
| Cera Camauba | 1,5 | 2 | 2 | 1,5 |
| Cera Alba | 0,5 | 0,5 | 0,5 | 0,5 |
| C16-40 Alkyl Stearat | | 1,5 | 1,5 | 1,5 |

(fortgesetzt)

| | 1 | 2 | 3 | 4* |
|---|---|---|---|---|
| Diethylhexyl-2,6-naphthalat | 5,5 | 13,0 | 2,5 | 8,0 |
| Butyl Methoxydibenzoylmethan | | 1 | 1 | |
| Z-Cote® HP1 | | | | 4,5 |
| MT-100 TV | | 4 | 2,5 | |
| 4-Methylbenzylidene Campher | | 3,6 | | 5 |
| Ethylhexyl Methoxycinnamat | 3 | 3,6 | 7,5 | 2,5 |
| Ansio Triazin | 2,5 | | | 5 |
| Octocrylene | | | 7,5 | |
| Benzophenone-3 | | | 3,5 | |
| Ethylhexyl Triazon | 2 | | | |
| Diethylhexyl Butamido Triazon | | | | 3 |
| Tocopheryl Acetat | 0,5 | 1 | 1 | 1 |
| Tocopherol; Ascorbyl Palmitat | 0,05 | 0,05 | 0,05 | 0,05 |
| Buxus Chinensis | 2 | 1 | 1 | 1 |
| Parfum, BHT | q.s | q.s | q.s | q.s |
| Ricinus Communis | ad.100 | ad.100 | ad.100 | ad.100 |

**6. PIT-Emulsionen**

| | 1* | 2 | 3 | 4 | 5 | 6 | 7* | 8 |
|---|---|---|---|---|---|---|---|---|
| Glycerinmonostearat SE | 0,50 | 2,00 | 3,00 | 5,00 | | | 0,50 | 4,00 |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | | 3,50 |
| Ceteareth-20 | | | | 2,00 | | 2,50 | 3,00 | |
| PEG-100 Stearat | 5,00 | | 1,00 | | 1,00 | | | 0,50 |
| Cetyl Alkohol | 2,50 | 1,00 | | 1,50 | | 0,50 | 1,50 | |
| Cetyl Palmitat | | | | 0,50 | | 1,00 | | |
| Cetyl Dimethicon Copolyol | 0,50 | | | | 0,50 | | 1,00 | |
| Polyglyceryl-2 Dipolyhydroxystearat | | | | 0,75 | 0,25 | | | |
| Diethylhexyl-2,6-naphthalat | 7,0 | 3,5 | 8,00 | 6,0 | 15,0 | 4,0 | 5,0 | 4,5 |
| Aniso Triazin | | | 0,50 | 2,00 | | 3,00 | | |
| Butyl Methoxydibenzoylmethan | 1,50 | | 1,00 | | 5,00 | 1,00 | 0,75 | |
| Bisimidazylat | | 2,00 | | | 1,00 | | | |
| Terephthaliden Dicampher Sulfonsäure | | | 0,50 | | | | 1,00 | |
| Drometrizol Trisiloxan | | | 2,00 | | | 3,00 | | 1,00 |

(fortgesetzt)

| | 1* | 2 | 3 | 4 | 5 | 6 | 7* | 8 |
|---|---|---|---|---|---|---|---|---|
| Ethylhexyl Methoxycinnamat | 8,00 | | | 4,50 | 5,00 | 8,00 | | |
| Ethylhexyl Salicylat | 4,00 | | | | 3,50 | 4,00 | | |
| Dioctyl Butamidotriazon | | | | 3,00 | 2,00 | 2,00 | | 1,50 |
| Ethylhexyl Triazon | | | 2,00 | 4,00 | | | 1,50 | 3,00 |
| Dimethicon Diethylbenzalmalonat | | 4,50 | | | 3,50 | | | |
| Octocrylen | | | 5,00 | | 8,00 | | | 7,50 |
| Phenylbenzmidazol Sulfonsäure | 1,00 | 5,00 | | 3,00 | 1,00 | | | |
| C12-15 Alkyl Benzoat | 3,50 | | | | 6,50 | 4,00 | | |
| Cocoglyceride | | 3,00 | | 3,00 | | 2,50 | | 3,50 |
| Dicaprylyl Ether | 4,00 | | | | | | | |
| Butylenglycol Dicaprylat/ Dicaprat | | 4,00 | | 3,00 | | | | |
| Dicaprylyl Carbonat | | | | 0,50 | | | | 6,00 |
| Dibutyl Adipate | | | 2,50 | | | 3,00 | | 1,00 |
| Phenyltrimethicon | 2,00 | | | | | 3,00 | | |
| Cyclomethicon | | 3,00 | | | | | | 4,00 |
| Ethyl Galaktomannan (N-Hance® AG-200) | | 0,50 | 3,50 | | 2,00 | | | |
| Hydrierte Coco-Glyceride | | | | 3,00 | 4,00 | | | 2,50 |
| Abil® Wax 2440 | | | | | | 1,50 | 3,00 | |
| PVP Hexadecen Copolymer | | | | 1,00 | 1,50 | | | |
| Glycerin | 10,0 | 5,00 | | 7,50 | | | | |
| Tocopherol | 1,00 | | | 0,75 | 0,50 | | 1,00 | |
| Shea Butter | | 2,00 | 3,50 | | | | | 0,50 |
| Iodopropyl Butylcarbamat | 0,12 | | | | 0,20 | 0,15 | | |
| DMDM Hydantoin | | | | 0,10 | | | | |
| Methylparaben | | 0,50 | 0,25 | | 0,45 | | | |
| Phenoxyethanol | 0,50 | 0,40 | | 1,00 | | | | 1,00 |
| Octoxyglycerin | | 0,30 | | | 1,00 | | | |
| Ethanol | | | | 2,00 | | | 7,50 | 4,00 |
| Trisodium EDTA | | 0,40 | | 0,15 | | 0,20 | | 0,50 |
| Parfüm | 0,20 | | 0,20 | 0,20 | 0,45 | | | 0,20 |
| Wasser | ad. 100 | ad. 100 | ad. 100 | ad. 100 | ad. 100 | ad. 100 | ad. 100 | ad. 100 |
| Die mit "*" gekennzeichneten Beispiele fallen nicht unter die Ansprüche. | | | | | | | | |

**Patentansprüche**

**1.** Kosmetische Zubereitung mit mindestens einem oxidations- und/oder UV-empfindlichen Wirkstoff, **dadurch ge-**

**kennzeichnet, dass** sie

(a) mindestens ein Dialkylnaphthalat, welches sich durch die Strukturformel

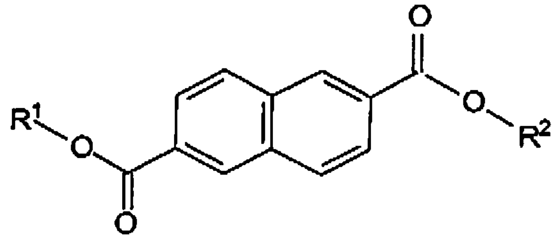

.

auszeichnet,
worin $R^1$ und $R^2$ unabhängig voneinander gewählt werden aus der Gruppe der verzweigten und unverzweigten Alkylgruppen mit 6 bis 24 Kohlenstoffatomen, und
(b) mindestens ein Wachs, welches aus der Gruppe der Triglyceride gewählt wird,

enthält.

**2.** Zubereitung nach Anspruch 1, **dadurch gekennzeichnet, dass** der Gehalt an einem oder mehreren Dialkylnaphthalaten aus dem Bereich von 0,001 bis 30 Gew.-%, vorteilhaft 0,01 bis 20 Gew.-%, besonders bevorzugt 1 bis 15 Gew.-% gewählt wird, jeweils bezogen auf das Gesamtgewicht der Zubereitung.

**3.** Zubereitung nach einem der Ansprüche 1 oder 2, **dadurch gekennzeichnet, dass** der oxidations- und/oder UV-empfindliche Wirkstoff 4-(tert.-Butyl)-4'-methoxydibenzoylmethan ist

**4.** Zubereitung nach einem der vorhergehenden Ansprüche, **dadurch gekennzeichnet, dass** der oder die oxidations- und/oder UV-empfindliche(n) Wirkstoff(e) aus der Gruppe der lipophilen Wirkstoffe gewählt wird/werden.

**5.** Zubereitung nach einem der vorhergehenden Ansprüche, **dadurch gekennzeichnet, dass** der oder die oxidations- und/oder UV-empfindliche(n) Wirkstoff(e) aus der folgenden Gruppe gewählt wird/werden:

- Coenzym Q10,
- Vitamin A und Derivate,
- Vitamin E und Derivate,
- Liponsäure und Derivate,
- Carotinoide.

**6.** Zubereitung nach einem der vorhergehenden Ansprüche, **dadurch gekennzeichnet, dass** sie mindestens eine UV-Filtersubstanz, gewählt aus der Gruppe Triazine, Benzotriazole, der bei Raumtemperatur flüssigen UV-Filter und organische und/oder anorganische Pigmente, enthält.

**7.** Zubereitung nach einem der vorhergehenden Ansprüche, **dadurch gekennzeichnet, dass** sie mindestens eine UV-A-Filtersubstanz und/oder einen Breitbandfilter, gewählt aus der Gruppe 2,4-Bis-{[4-(2-Ethyl-hexyloxy)-2-hydroxy]-phenyl}-6-(4-methoxyphenyl)-1,3,5-triazin, Phenylen-1,4-bis-(2-benzimidazyl)-3,3'-5,5'-tetrasulfonsäure-bis-natriumsalz, Benzol-1,4-di(2-oxo-3-bornylidenmethyl-10-sulfonsäure, 2-(2H-benzotriazol-2-yl)-4-methyl-6-[2-methyl-3-[1,3,3,3-tetramethyl-1-[(trimethylsilyl)oxy]disiloxanyl]propyl]-phenol, enthält, wobei die weiteren Filtersubstanzen jeweils einzeln oder in beliebigen Kombinationen miteinander vorliegen können.

**8.** Zubereitung nach einem der vorhergehenden Ansprüche, **dadurch gekennzeichnet, dass** sie mindestens ein Flavonglycosid, insbesondere α-Glucosylrutin, und/oder Vitamin E und/oder dessen Derivate enthält.

**9.** Verwendung von Zubereitungen nach einem der Ansprüche 1 bis 8 zum Schutz vor lichtbedingter Hautalterung.

**10.** Verwendung von Stoffkombinationen, welche

(a) mindestens ein Dialkylnaphthalat, welches sich durch die Strukturformel

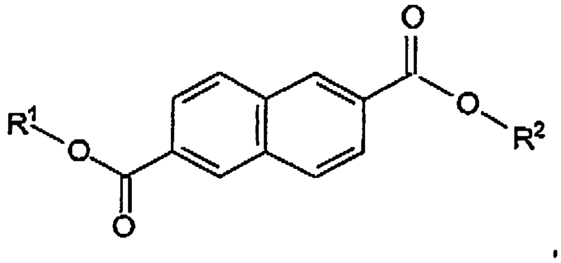

auszeichnet,
worin $R^1$ und $R^2$ unabhängig voneinander gewählt werden aus der Gruppe der verzweigten und unverzweigten Alkylgruppen mit 6 bis 24 Kohlenstoffatomen, und
(b) mindestens ein Wachs, welches aus der Gruppe der Triglyceride gewählt wird, enthalten,

zur Stabilisierung kosmetischer oder dermatologischer Wirkstoffe gegen die durch UV-Strahlung induzierte Zersetzung.

**11.** Verwendung von Stoffkombinationen, welche

(a) mindestens ein Dialkylnaphthalat, welches sich durch die Strukturformel

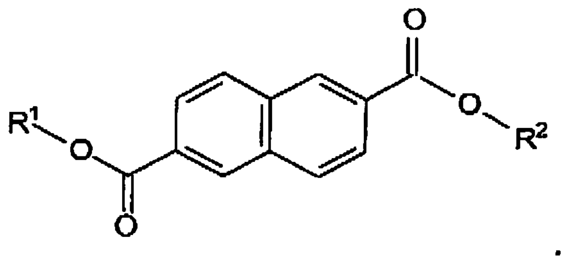

auszeichnet,
worin $R^1$ und $R^2$ unabhängig voneinander gewählt werden aus der Gruppe der verzweigten und unverzweigten Alkylgruppen mit 6 bis 24 Kohlenstoffatomen, und
(b) mindestens ein Wachs, welches aus der Gruppe der Triglyceride gewählt wird, enthalten, zur Verbesserung der Wirksamkeit und zur Erhöhung der Stabilität von lipophilen Wirkstoffen in kosmetischen oder dermatologischen Zubereitungen.

## Claims

**1.** Cosmetic preparation containing at least one oxidation- and/or UV-sensitive active ingredient, **characterized in that** it comprises

(a) at least one dialkyl naphthalate which is **characterized by** the structural formula

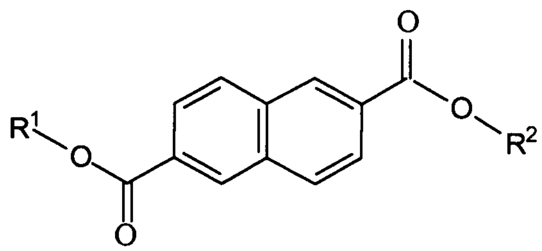

,

in which $R^1$ and $R^2$, independently of one another, are chosen from the group of branched and unbranched alkyl groups having 6 to 24 carbon atoms,
and
(b) at least one wax which is chosen from the group of triglycerides.

**2.** Preparation according to Claim 1, **characterized in that** the content of one or more dialkyl naphthalates is chosen from the range from 0.001 to 30% by weight, advantageously 0.01 to 20% by weight, particularly preferably 1 to 15% by weight, in each case based on the total weight of the preparation.

**3.** Preparation according to either of Claims 1 and 2, **characterized in that** the oxidation- and/or UV-sensitive active ingredient is 4-(tert-butyl)-4'-methoxydibenzoylmethane.

**4.** Preparation according to one of the preceding claims, **characterized in that** the oxidation- and/or UV-sensitive active ingredient(s) is/are chosen from the group of lipophilic active ingredients.

**5.** Preparation according to one of the preceding claims, **characterized in that** the oxidation- and/or UV-sensitive active ingredient(s) is/are chosen from the following group:

(a) coenzyme Q10,
(b) vitamin A and derivatives,
(c) vitamin E and derivatives,
(d) lipoic acid and derivatives,
(e) carotenoids.

**6.** Preparation according to one of the preceding claims, **characterized in that** it comprises at least one UV filter substance chosen from the group consisting of triazines, benzotriazoles, the UV filters liquid at room temperature and organic and/or inorganic pigments.

**7.** Preparation according to one of the preceding claims, **characterized in that** it comprises at least one UV-A filter substance and/or a broadband filter chosen from the group consisting of 2,4-bis{[4-(2-ethylhexyloxy)-2-hydroxy]phenyl}-6-(4-methoxyphenyl)-1,3,5-triazine, phenylene-1,4-bis(2-benzimidazyl)-3,3'-5,5'-tetrasulfonic acid bis-sodium salt, benzene-1,4-di(2-oxo-3-bornylidenemethyl-10-sulfonic acid, 2-(2H-benzotriazol-2-yl)-4-methyl-6-[2-methyl-3-[1,3,3,3-tetramethyl-1-[(trimethylsilyl)oxy]disiloxanyl]propyl]phenol, where the further filter substances may in each case be present individually or in any combinations with one another.

**8.** Preparation according to one of the preceding claims, **characterized in that** it comprises at least one flavone glycoside, in particular $\alpha$-glucosylrutin, and/or vitamin E and/or derivatives thereof.

**9.** Use of preparations according to one of Claims 1 to 8 for protection against photoinduced skin aging.

**10.** Use of substance combinations which comprise

(a) at least one dialkyl naphthalate which is **characterized by** the structural formula

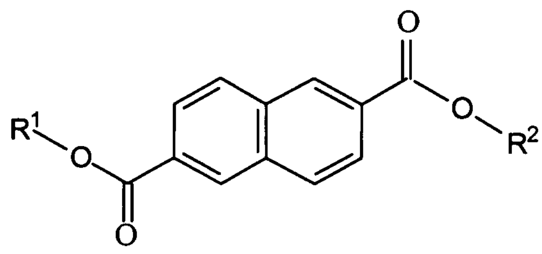

,

in which $R^1$ and $R^2$, independently of one another, are chosen from the group of branched and unbranched alkyl groups having 6 to 24 carbon atoms,
and
(b) at least one wax which is chosen from the group of triglycerides
for stabilizing cosmetic or dermatological active ingredients against decomposition induced by UV radiation.

**11.** Use of substance combinations which comprise

(a) at least one dialkyl naphthalate which is **characterized by** the structural formula

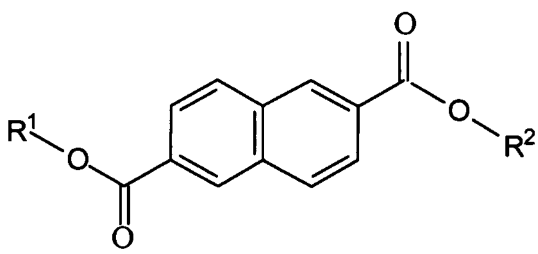

,

in which $R^1$ and $R^2$, independently of one another, are chosen from the group of branched and unbranched alkyl groups having 6 to 24 carbon atoms, and
(b) at least one wax which is chosen from the group of triglycerides
for improving the effectiveness and increasing the stability of lipophilic active ingredients in cosmetic or dermatological preparations.

## Revendications

**1.** Préparation cosmétique comportant au moins une substance active sensible à l'oxydation et/ou aux UV, **caractérisée en ce qu'**elle contient

• au moins un naphtalate de dialkyle qui se distingue par la formule développée

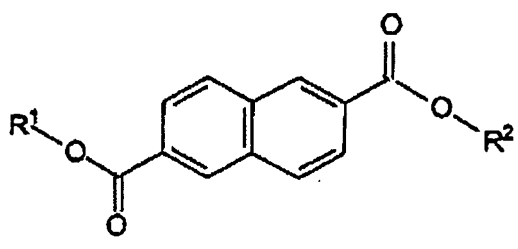

,

dans laquelle $R^1$ et $R^2$ sont choisis, indépendamment l'un de l'autre, dans l'ensemble des groupes alkyle ramifiés ou non ramifiés ayant de 6 à 24 atomes de carbone
et

- au moins une cire, qui est choisie dans le groupe des triglycérides.

**2.** Préparation selon la revendication 1, **caractérisée en ce que** la teneur en un ou plusieurs naphtalates de dialkyle est choisie dans la plage allant de 0,001 à 30 % en poids, avantageusement de 0,01 à 20 % en poids, de façon particulièrement préférée de 1 à 15 % en poids, chaque fois par rapport au poids total de la préparation.

**3.** Préparation selon la revendication 1 ou 2, **caractérisée en ce que** la substance active sensible à l'oxydation et/ou aux UV est le 4-(tert-butyl)-4'-méthoxydibenzoylméthane.

**4.** Préparation selon l'une quelconque des revendications précédentes, **caractérisée en ce que** la ou les substance (s) active(s) sensible(s) à l'oxydation et/ou aux UV est/sont choisie(s) dans le groupe des substances actives lipophiles.

**5.** Préparation selon l'une quelconque des revendications précédentes, **caractérisée en ce que** la ou les substance (s) active(s) sensible(s) à l'oxydation et/ou aux UV est/sont choisie(s) dans le groupe suivant :

- coenzyme Q10,
- vitamine A et dérivés,
- vitamine E et dérivés,
- acide lipoïque et dérivés,
- caroténoïdes.

**6.** Préparation selon l'une quelconque des revendications précédentes, **caractérisée en ce qu'**elle contient au moins un filtre UV, choisi dans l'ensemble constitué par les triazines, les benzotriazoles, les filtres UV liquides à la température ambiante et les pigments organiques et/ou minéraux.

**7.** Préparation selon l'une quelconque des revendications précédentes, **caractérisée en ce qu'**elle contient au moins un filtre UV-A et/ou un filtre à large bande, choisi dans l'ensemble constitué par la 2,4-bis{[4-(2-éthyl-hexyloxy)-2-hydroxy]-phényl}-6-(4-méthoxyphényl)-1,3,5-triazine, le phénylène-1,4-bis(2-benzimidazyl)-3,3',5,5'-tétrasulfonate disodique, l'acide benzène-1,4-di(2-oxo-3-bornylidèneméthyl-10-sulfonique, le 2-(2H-benzotriazol-2-yl)-4-méthyl-6-[2-méthyl-3-[1,3,3,3-tétraméthyl-1-[(triméthylsilyl)oxy]disiloxanyl]propyl]-phénol, les autres filtres pouvant être présents chacun individuellement ou en associations quelconques entre eux.

**8.** Préparation selon l'une quelconque des revendications précédentes, **caractérisée en ce qu'**elle contient au moins un flavone-glycoside, en particulier de l'α-glucosylrutine, et/ou de la vitamine E et/ou des dérivés de celle-ci.

**9.** Utilisation des préparations selon l'une quelconque des revendications 1 à 8, pour la protection contre le vieillissement photo-induit de la peau.

**10.** Utilisation d'associations de substances qui contiennent

(a) au moins un naphtalate de dialkyle qui se distingue par la formule développée

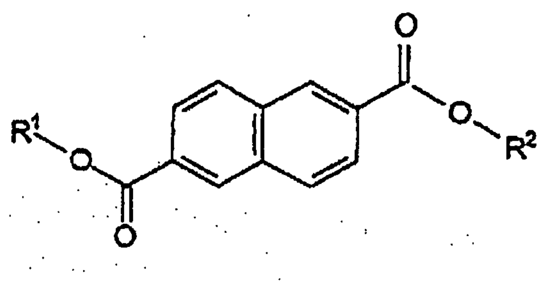

dans laquelle $R^1$ et $R^2$ sont choisis, indépendamment l'un de l'autre, dans l'ensemble des groupes alkyle ramifiés et non ramifiés ayant de 6 à 24 atomes de carbone et
(b) au moins une cire, qui est choisie dans le groupe des triglycérides,
pour la stabilisation de substances actives cosmétiques ou dermatologiques contre la décomposition induite par le rayonnement UV.

**11.** Utilisation d'associations de substances qui contiennent

(a) au moins un naphtalate de dialkyle qui se distingue par la formule développée

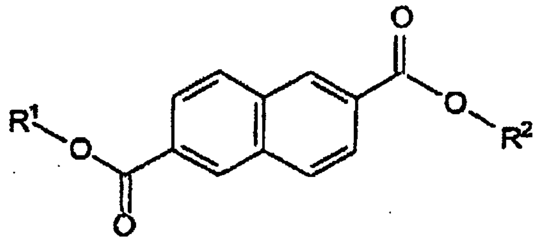

dans laquelle $R^1$ et $R^2$ sont choisis, indépendamment l'un de l'autre, dans l'ensemble des groupes alkyle ramifiés et non ramifiés ayant de 6 à 24 atomes de carbone et
(b) au moins une cire, qui est choisie dans le groupe des triglycérides,
pour l'amélioration de l'efficacité et l'augmentation de la stabilité de substances actives lipophiles dans des préparations cosmétiques ou dermatologiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- XP 001010090
- FR 2801206
- FR 2801209
- EP 1127569 A
- US 5663213 A
- EP 0761201 A


### In der Beschreibung aufgeführte Nicht-Patentliteratur


- **FETTE ; SEIFEN.** *Anstrichmittel,* 1974, vol. 76, 135